# EUROPÄISCHE PATENTSCHRIFT

(11) **EP 3 410 983 B1**
(45) Veröffentlichungstag und Bekanntmachung des Hinweises auf die Patenterteilung: **07.04.2021**
(21) Anmeldenummer: 17703133.3
(22) Anmeldetag: 03.02.2017
(51) Int. Cl.: A61F 2/07, A61F 2/89, A61F 2/06

(54) **GEFÄSSPROTHESE MIT SEITENÄSTEN**
VASCULAR PROSTHESIS HAVING SIDE BRANCHES
PROTHÈSE VASCULAIRE À BRANCHES LATÉRALES

(30) Priorität: 04.02.2016 DE 102016102008
(43) Veröffentlichungstag der Anmeldung: 12.12.2018
(73) Patentinhaber: JOTEC GmbH, 72379 Hechingen (DE)
(72) Erfinder: WALTHER, Michael, 72108 Rottenburg (DE); KLEIN, Karsten, 72116 Moessingen (DE); MERZ, Juergen, 72336 Balingen (DE); ULMER, Jan, 71067 Sindelfingen (DE)
(74) Vertreter: Witte, Weller & Partner Patentanwälte mbB
(86) Internationale Anmeldenummer: PCT/EP2017/052336
(87) Internationale Veröffentlichungsnummer: WO 2017/134198

(56) Entgegenhaltungen:
- EP-A1- 2 606 851
- EP-A1- 2 740 441
- WO-A1-2015/109375
- DE-B3-102012 101 103
- US-A1- 2014 094 902

## Beschreibung

Die vorliegende Erfindung betrifft eine verzweigte Gefäßprothese für ein Blutgefäß eines Patienten.

Allgemein ist es bekannt, intraluminale Gefäßprothesen oder - implantate, die auch als endovaskuläre Gefäßstents/-stentgrafts bezeichnet werden, zur Behandlung von geschwächten, verletzten, gerissenen oder aneurysmatischen Gefäßen einzusetzen. Hierfür wird an der erkrankten oder verletzten Stelle des Gefäßes ein Gefäßimplantat bzw. Stentgraft freigesetzt, der die Funktionalität des ursprünglichen Gefäßes wieder herstellt bzw. die noch bestehende Gefäßintegrität unterstützt.

Dabei versteht man unter einem Aneurysma eine Ausweitung oder Aussackung eines arteriellen Blutgefäßes infolge angeborener oder erworbener Wandveränderungen. Die Aussackung kann dabei die Gefäßwand als Ganzes erfassen, oder, wie bei dem sogenannten falschen Aneurysma bzw. der sogenannten Dissektion, es tritt Blut aus dem Lumen des Gefäßes zwischen die Gefäßwandschichten und schert diese auseinander. Die Nichtbehandlung eines Aneurysma kann im fortgeschrittenen Stadium zu einer Ruptur der Arterie führen, mit der Folge, dass der Patient innerlich verblutet. Ursache eines thorakalen und thorakoabdominalen Aortenaneurysmas können Arteriosklerose, Bluthochdruck sowie Entzündungsprozesse der Gefäßwand sein. Auch Verletzungen des Brustkorbes durch schwere Unfälle können akut oder chronisch zu einem Aortenaneurysma führen.

Die zur Behandlung von Aneurysmen verwendeten selbstexpandierenden Gefäßprothesen bestehen im Allgemeinen aus einem hohlzylindrischen Metallrahmen bzw.-gerüst, dessen Mantelfläche mit einer Textil- oder Polymerfolie abgedeckt ist, so dass sich ein hohlzylindrischer Körper ergibt. Zur Implantation wird das Gefäßimplantat radial zusammengedrückt, so dass sich seine Querschnittsfläche deutlich verringert. Das Gefäßimplantat wird dann mit Hilfe eines Einführsystems in den Bereich des Aneurysma gebracht, wo es freigesetzt wird. Auf Grund der Federwirkung des Metallrahmens/-gerüsts expandiert das Gefäßimplantat wieder in seine ursprüngliche Form und spannt dabei seine Mantelfläche auf, die sich proximal und distal von dem Aneurysma innen in dem Blutgefäß verklemmt. Auf diese Weise fließt das Blut jetzt durch das Gefäßimplantat und eine weitere Belastung der Aussackung wird verhindert.

Der Metallrahmen solcher Gefäßimplantate besteht in der Regel bspw. aus einem Drahtgeflecht oder aus hintereinander angeordneten, mäanderförmig umlaufenden, sogenannten Stentfedern, die ggf. über Verbindungsstützen aus Draht miteinander verbunden sind, oder die lediglich über das Implantatmaterial miteinander verbunden sind. Das Drahtgeflecht bzw. die Stentfedern sind üblicherweise aus einem Shape-Memory-Material, in der Regel aus Nitinol, wodurch die Stentfedern nach Einbringung in ein Gefäß zur Freisetzung wieder in den expandierten Zustand übergehen und das Gefäßimplantat dadurch "aufspannen".

Aneurysmen treten häufig im Bereich der Baucharterie (Aorta abdominalis) oder Brustarterie (Aorta thoracica) auf, wobei ein thorakales Aneurysma im sogenannten aufsteigenden Ast der Aorta (Aorta ascendens), im Aortenbogen und/oder im absteigenden Ast der Aorta auftreten kann.

Beim thorakoabdominellen Aortenaneurysma bleibt das Aneurysma nicht nur auf einen begrenzten Teil der Hauptschlagader begrenzt, sondern findet sich sowohl im Brustraum, also im Thorax als auch im Bauchraum (Abdomen).

Das thorakoabdominelle Aortenaneurysma ist ein sehr komplexex Krankheitsbild und schwierig zu behandeln. Vor der Etablierung von Aortenstents war es nahezu immer notwendig, sowohl Brustkorb als auch Bauchraum zu öffnen. Heute kann alternativ, im Rahmen von Hybrid-Operationen, auch eine Eröffnung der Bauchhöhle erfolgen in Kombination mit einem Aortenstent, bzw. eine totale endovaskuläre Behandlung von thorakoabdominellen Aortenaneurysmen.

Erschwerend kommt beim thorakoabdominellen Aortenaneurysma meist immer hinzu, dass sämtliche wichtigen Organarterien (Darmarterien, Truncus coeliacus, Nierenarterien) bei der Reparation mit betroffen sind und in irgendeiner Form rekonstruiert werden müssen. Daher ist die Therapie des thorakoabdominellen Aneurysmas schwierig, mit Komplikationen behaftet, und wird regelmäßig von speziellen Zentren durchgeführt.

Die bei der Therapie eingesetzten Gefäßprothesen müssen dabei viele Erfordernisse erfüllen, insbesondere im Hinblick auf die Größe, Länge und Morphologie des zu überbrückenden Aneurysmas und der nativen Aorta; ferner spielt die Durchlässigkeit der Segmentarterien in diesem Bereich, die Koexistenz weiterer Aneurysmen und der Durchmesser der Iliacal- und Femoralgefäße eine große Rolle.

Die EP 2 740 441 A1 offenbart einen Stentgraft zur Freisetzung in der Aorta eines Patienten, mit einem röhrchenförmigen Körper und mit vom Körper außen abgehenden kurzen Seitenästen in einem mittleren Bereich des Körpers.

Die US 2014/0094902 A1 offenbart ebenfalls einen Endograft mit einem röhrenförmigen Grundkörper, der außen hiervon abgehende Seitenäste aufweist.

Die WO 2015/109375 A1 schließlich offenbart eine Endoprothese mit einem röhrchenförmigen Grundkörper, der in einem mittleren Bereich elliptische Öffnungen für Seitengefäße aufweist.

Nach wie vor besteht daher ein großes Bedürfnis nach Stent-/Stentgraftsystemen, bzw Gefäßprothesen, mit Hilfe derer der oben geschilderte Eingriff vereinfacht und zeitlich verkürzt werden könnte.

Aufgabe der vorliegenden Erfindung ist es daher, ein System bereitzustellen, mit welchem thorakoabdominelle Aneurysmen schnell und unkompliziert behandelt werden können.

Erfindungsgemäß wird diese Aufgabe gelöst durch eine vorzugswiese selbstexpandierende Gefäßprothese mit einem hohlzylindrischen Grundkörper mit einer Längsachse, einer proximalen und einer distalen Öffnung, sowie einem sich zwischen der proximalen und der distalen Öffnung erstreckenden Hauptlumen, wobei der Grundkörper einen proximalen und einen distalen Endabschnitt aufweist, der jeweils die erste oder die zweite Öffnung umfasst, sowie einem zwischen dem proximalen und dem distalen Endabschnitt angeordneten mittleren Abschnitt; ferner mit einer Vielzahl an nicht untereinander verbundenen Stentringen, die in Längsrichtung der Gefäßprothese mäanderförmig umlaufend und in einem Abstand hintereinander angeordnet sind, wobei die Stentringe jeweils Spitzbögen und diese verbindenden Stege aufweisen; ferner mit einem Prothesenmaterial, an welches die Stentringe angebracht sind, und ferner mit zumindest vier Seitenästen, die sich von zwischen den Stentringen liegenden Fenestrierungen im Prothesenmaterial ins Hauptlumen nach proximal erstrecken, wobei jeder Seitenast eine erste und eine zweite Seitenast-Öffnung sowie ein sich zwischen der ersten und der zweiten Seitenast-Öffnung erstreckendes Seitenast-Lumen aufweist, wobei die erste Seitenast-Öffnung jeweils fest an das Prothesenmaterial über jeweils einer Fenestrierung angebracht ist zur Ausbildung eines Seitenast-Ansatzes; die erfindungsgemäße Gefäßprothese weist ferner vier Seitenäste auf, die sich ins Innere des Hauptlumens erstrecken, und im mitterlen Abschnitt angeordnet sind, wobei der mittlere Abschnitt einen im Vergleich zum Durchmesser der Endabschnitte kleineren Durchmesser aufweist.

Die der Erfindung zugrunde liegende Aufgabe wird auf diese Weise vollkommen gelöst.

Mit der erfindungsgemäßen selbstexpandierenden Gefäßprothese wird ein Gefäßprothesen-System bereitgestellt, mit dem thorakoabdominelle Aneurysmen erfolgreich überbrückt werden können, wobei gleichzeitig die Versorgung der abgehenden Seitengefäße, also bspw. der Viszeralarterien, sicher gewährleistet wird. Dies wird dadurch erreicht, dass bei der erfindungsgemäßen Gefäßprothese die Seitenäste/Ärmchen, über welche die Seitengefäße, insbesondere durch Anschließen weiterer Verbindungs-Stents/-Stentgrafts, sicher versorgt werden, im Inneren des Hautplumens der Gefäßprothese geführt sind. Durch den nach innen geführten Abgang der Seitenäste/Ärmchen wird gewährleistet, dass in dem Gefäß, in welchem die Gefäßprothese freigesetzt wird, hinreichend Platz für die Gefäßprothese ist, wobei gleichzeitig die Funktion der Ärmchen/Seitenäste gewährleistet wird. Gegenüber reinen Fenestrierungen haben verzweigte Gefäßprothesen wie die vorliegende den Vorteil einer längeren Überlappungszone mit Verbindungs-Stents/-Stentgrafts für die abzweigenden Seitengefäße.

Die erfindungsgemäße Gefäßprothese hat überdies den Vorteil, dass durch die spezifische Anordnung der nach Innen ins Hauptlumen abgehenden Seitenäste ein sogenanntes "off-the-shelf"-System bereitgestellt wird, mithin also eine Gefäßprothese, die nicht jeweils individuell auf den spezifisch zu behandelnden Patienten angefertigt werden muss, sondern die aufgrund ihrer spezifischen Anordnung und Dimensionen der Seitenäste gerade für eine Vielzahl an Patienten vorgefertigt werden kann.

Alle vier Seitenäste der erfindungsgemäßen Gefäßprothese sind im mittleren Abschnitt angeordnet, der einen kleineren Durchmesser aufweist als die beiden rechts und links, bzw. proximal und distal, vom mittleren Abschnitt angeordneten Abschnitte.

Die erfindungsgemäße Gefäßprothese ans sich bzw. der Grundkörper ist dabei einstückig ausgebildet, d.h. die Stentringe, ggf. mit unterschiedlichen Durchmessern, sind in Abständen zueinander an dem Prothesenmaterial angebracht, bspw. angenäht. Die Stentringe können dabei außen oder innen an das Prothesenmaterial angenäht sein.

Die Seitenäste umfassen vorzugsweise einen bzw. zumindest einen Stentring, der an ein Prothesenmaterial - von innen oder von außen - angebracht ist.

Es versteht sich, dass die Seitenäste, die vorliegend und im Stand der Technik auch als "Ärmchen" bzw. "Manschetten" bezeichnet werden, sich dabei nicht über die gesamte Länge der Gefäßprothese erstrecken, sondern nur kurze Stentgraftabschnitte darstellen, mit welchen ein Verbindungsstück für anzuschließende Stent/Stentgrafts bereitgestellt wird, die in die Seitengefäße abgehen.

Grundsätzlich werden bei Gefäßprothesen oder endoluminalen Stentgrafts allgemein sowie vorliegend zur Bezeichnung der jeweiligen Enden die Begriffe "distal" und "proximal" verwendet, wobei der Begriff "distal" der Teil bzw. das Ende bezeichnet wird, der/das in Bezug auf den Blutstrom weiter stromabwärts liegt. Der Begriff "proximal" hingegen bezeichnet, wieder in Bezug auf den Blutstrom, einen Teil bzw. das Ende, der/das in Bezug auf den Blutstrom weiter stromaufwärts liegt. Anders ausgedrückt bedeutet der Begriff "distal" in Richtung des Blutstroms, und der Begriff "proximal" der Richtung des Blutstroms entgegengesetzt. Bei Kathetern hingegen, bzw. Einführsystemen bezeichnet der Begriff "distal" das Ende des Katheters bzw. Einführsystems, das in den Patienten eingeführt wird, bzw. das vom Anwender ausgesehen am Entferntesten liegt, und der Begriff "proximal" das Ende, das sich dem Anwender näher zugewandt ist.

Erfindungsgemäß bedeutet die Einteilung des Grundköpers der Gefäßprothese in proximaler Endabschnitt, mittlerer Abschnitt und distaler Endabschnitt, dass sich die jeweiligen Abschnitte durch eine voneinander unterschiedlichen Bauweise unterscheiden, und bspw. eine unterschiedliche Anzahl an Stentringen aufweisen, bzw. deren Stentringe unterschiedliche Durchmesser aufweisen, so dass sich hieraus eine unterschiedliche äußere Form der Abschnitte mit unterschiedlichen Durchmessern des Grundköpers in diesen Abschnitten ergibt,

"Fenestrierung" bedeutet vorliegend, bzw. allgemein im Stand der Technik, dass das eine Mantelfläche aufspannende Prothesenmaterial in seiner Mantelfläche eine bzw. zumindest eine Öffnung, sozusagen ein Loch, aufweist, wodurch ein Zugang zum Hauptlumen, also zum Inneren des Hauptlumens geschaffen wird. Erfindungsgemäß werden diese Fenestrierungen nach innen, also ins Hauptlumen durch die Seitenäste verlängert.

In einer bevorzugten Ausführungsform der erfindungsgemäßen Gefäßprothese weisen alle Stentringe im mittleren Bereich den gleichen Durchmesser auf.

In noch einer bevorzugten Ausführungsform weisen zusätzlich sowohl der proximale Endabschnitt als auch der distale Endabschnitt jeweils einen sich zum mittleren Abschnitt im Durchmesser verjüngenden Abschnitt auf.

Gemäß einer Ausführungsform der erfindungsgemäßen Gefäßprothese sind die die Spitzbögen verbindenden Stege der Stentringe im mittleren Bereich kürzer sind als die Stege, die die Spitzbögen der Stentringe in den beiden Endabschnitten verbunden.

Diese Ausführungsform hat den Vorteil, dass dadurch die Stentringe im mittleren Bereich einen engeren Durchmesser aufweisen, und zwar aufgrund der kürzeren Ausbildung der Stege. In anderen Worten, weisen dadurch die Stentringe im mittleren Abschnitt, bei gleichbleibender Anzahl der Spitzbögen, einen kleineren Durchmesser auf als die Stentringe im distalen bzw. proximalen Abschnitt.

In einer weiteren Ausführungsform weisen die Stege aller Stentringe im mittleren Bereich die gleiche Länge auf. Dies bedeutet, dass die Stentringe im mittleren Bereich alle den gleichen, bzw. im Wesentlichen gleichen, Durchmesser haben, und sich von den Stentringen des proximalen und distalen Abschnitts in ihrem Durchmesser unterscheiden.

Vorzugswiese weist eine Ausführungsform der Gefäßprothese im mittleren Abschnitt 4, 5 oder 6 Stentringe auf, der proximale Abschnitt 3, 4, oder 5 Stentringe, und der distale Abschnitt 2, 3 oder vier Stentringe.

Die mäanderförmig umlaufenden Stentringe der erfindungsgemäßen Gefäßprothese sind vorzugsweise aus einem selbstexpandierendem Material mit Shape-Memory-Eigenschaft gefertigt, und sind vorzugsweise aus Nitinol, die abwechselnd in proximale und distale Richtung weisende Spitzbögen und diese verbindende Stege aufweisen. Auch die Seitenäste können auf diese Weise aufgebaut sein. Eine nähere Beschreibung solcher Stents sowie des Graft-/Prothesenmaterials findet sich bspw. in der DE 103 37 739, auf deren gesamten Inhalt hiermit spezifisch Bezug genommen wird. Die Stentringe sind an Prothesenmaterial gefestigt, wobei bevorzugt ist, wenn das Prothesenmaterial ein Material aufweist, das ausgewählt ist aus einem Textil oder einem Polymer.

Insbesondere ist bevorzugt, wenn das Prothesenmaterial ein Material aufweist oder aus diesem gebildet ist, das ausgewählt ist aus Polyester, Polyurethan, Polystyrol, Polytetrafluorethylen ultrahochmolekulargewichtiges Polyethylen (UHMPE), oder Mischungen davon.

In einer Weiterbildung der erfindungsgemäßen Gefäßprothese weist die erste Seitenast-Öffnung eines jeden Seitenasts, welche jeweils fest an das Prothesenmaterial über jeweils einer Fenestrierung angebracht ist, eine im Wesentlichen ovale Grundform aufweist.

Dabei bedeutet "im Wesentlichen", dass die Öffnung nicht exakt oval sein muss, sondern dass die Form der Öffnung eine derartige Form aufweist, die noch als oval aufgefasst wird. Diese Ausführungsform hat den Vorteil, dass der Abgang der Seitenäste ins Innere des Hauptlumens der Grundkörpers stark winklig sein kann, und zwar, gemäß einer Ausführungsform, praktisch parallel zu der Längsachse der Gefäßprothese.

Gemäß einer weiteren Ausführungsform der erfindungsgemäßen Gefäßprothese weist die zweite Seiten-Öffnung eines jeden Seitenasts über eine im Wesentlichen kreisrunde Grundform auf.

Mit dieser Ausführungsform wird erreicht, dass die freie, zweite Seitenast-Öffnung eine gleichmäßige Öffnung bietet, in die bspw. Verbindungs-Stentgrafts/- Stents einfach eingeführt werden können.

Gemäß einer Weiterbildung der erfindungsgemäßen Gefäßprothese sind die Seitenäste im mittleren Abschnitt derart angeordnet, dass der Seitenast-Ansatz eines ersten Seitenasts, in Bezug auf die Längsachse des Grundkörpers, zwischen 5 und 15 °, vorzugsweise um 10° verdreht ist, derart, dass der Seitenast in einem Winkel von 10° in Bezug auf die Längsachse in das Hauptlumen hineinragt, und dass die Seitenast-Ansätze der drei anderen Seitenäste in Bezug auf die Längsachse unverdreht sind. Dabei bedeutet "unverdreht", dass die unverdrehten Seitenäste im wesentlichen parallel zur Längsachse verlaufen, wohingegen "verdreht" bedeutet, dass der entsprechend verdrehte Seitenast, bzw. die verdrehte Seitenast-Öffnung, sich in einem Winkel von der Längsachse erstreckt. Da die erste Seitenast-Öffnung in einer bevorzugten Ausführungsform eine ovale Grundform hat versteht sich, dass die unverdrehte Anordnung des Seitenast-Ansatzes bedeutet, dass der längere Durchmesser der ovalen Grundform parallel zur Längsachse ist.

Mit der Verdrehung einer Seitenast-Öffnung im Verhältnis zur Längsachse des Grundkörpers wird erreicht, dass dieser in einem Winkel von der Längsachse der Gefäßprothese absteht; damit wird wiederum erreicht, dass die Seitenast-Öffnung des verdrehten Seitenasts nicht mit den anderen Seitenästen, bzw. dem nächstangeordenten, kollidiert. Die Handhabung und Beeinflussung der Seitenäste ist damit deutlich vereinfacht.

Gemäß einer Weiterbildung der erfindungsgemäßen Gefäßprothese sind im mittleren Abschnitt zwei Seitenäste weiter distal angeordnet als die zwei anderen, proximalen, Seitenäste. Entsprechend umfasst die erfindungsgemäße Gefäßprothese zwei am proximalsten angeordente Seitenäste und zwei am distalsten angeordnete Seitenäste.

Insgesamt ist bei der erfindungsgemäßen Gefäßprothese bevorzugt, wenn die Seitenäste bzw. deren erste Seitenast-Öffnung-Ansatz jeweils zwischen zwei Stentringen angeordnet sind,

Dabei ist bevorzugt, wenn alle Seitenäste verteilt und versetzt über den mittleren Abschnitt angeordnet sind, und zwar derart, dass der Seitenast-Ansatz der beiden am distalsten angeordenten Seitenäste, die beide distal zu den anderen beiden am proximalsten angeordenten Seitenästen liegen, zwischen denselben Stentringen angeordnet sind. Bevorzugt ist ferner, wenn der Seitenast-Ansatz des am proximalsten angeordenten Seitenasts und des diesem in distale Richtung nachgeordneten Seitenasts zwischen zwei unterschiedlichen Stentringen angeordnet sind.

Gemäß einer weiteren Ausführungsform der erfindungsgemäßen Gefäßprothese sind die Seitenast-Ansätze der proximalsten Seitenäste in Umfangsrichtung des hohlzylindrischen Grundkörpers versetzt zueinander angeordnet.

Diese Ausführungsform hat den Vorteil, dass die Seitenäste bzw. deren freie zweite Seitenast-Öffnung sich nicht überlappen und nicht miteinander kollidieren.

In einer Weiterbildung ist bevorzugt, wenn die Seitenast-Ansätze der beiden proximalsten Seitenäste gegenüber den Seitenast-Ansätzen der beiden am distalsten angeordneten Seitenäste in Umfangsrichtung versetzt zueinander angeordnet sind.

Diese Ausführungsform hat den Vorteil, dass alle vier Seitenäste sich im Hauptlumen nicht überlappen und damit auch nicht kollidieren, so dass deren Handhabung deutlich verbessert wird.

Gemäß einer bevorzugten Ausführungsform der erfindungsgemäßen Gefäßprothese sind der Seitenast-Ansatz des am proximalsten angeordneten Seitenasts und der Seitenast-Ansatz des diesem in distale Richtung angeordneten Seitenasts in einem Umfangswinkel von ca. 25° bis 35°, vorzugsweise von ca. 29° bis 32° versetzt zueinander angeordnet.

Gemäß einer weiteren Ausführungsform der erfindungsgemäßen Gefäßprothese sind der Seitenast-Ansatz der beiden am distalsten angeordneten Seitenäste in einem Umfangswinkel von ca. 140° bis 160°, vorzugsweise von ca. 149° bis ca. 152° versetzt zueinander angeordnet.

Dabei bedeutet "Umfangswinkel" vorliegend dass die Seitenast-Ansätze über den Umfang des hohlzylindrischen Grundkörpers in einem bestimmten Winkel zueinander verteilt angeordnet sind.

In einer weiteren Ausführungsform der erfindungsgemäßen Gefäßprothese sind der Seitenast-Ansatz des am proximalsten angeordneten Seitenasts und der Seitenast-Ansatz des diesem in distale Richtung nachgeordneten Seitenasts, bezogen auf einen jeweiligen Mittelpunkt der Seitenast-Ansätze, in Längsrichtung zwischen ca. 15 und 25 mm, vorzugsweise zwischen ca. 18 und 22 mm voneinander beabstandet.

Dabei bedeutet vorliegend der Begriff "ca.", dass die angegebenen Werte nicht immer exakt, d.h. auf die Nullstelle hinter dem Komma, verwirklicht werden müssen, sondern dass auch geringfügig abweichende Werte von bspw. 14,95 bis 15,05 mm umfasst sind.

In einer Weiterbildung der erfindungsgemäßen Gefäßprothese sind der Seitenast-Ansatz des am zweit proximalsten angeordneten Seitenasts und der Seitenast-Ansatz des diesem in distale Richtung nachgeordneten Seitenasts, bezogen auf einen jeweiligen Mittelpunkt der Seitenast-Ansätze, in Längsrichtung zwischen ca. 12 und 20 mm, vorzugsweise zwischen ca. 14 und 18 mm, voneinander beabstandet.

Ferner ist gemäß einer weiteren Ausführungsform bevorzugt, wenn der Seitenast-Ansatz des am dritt proximalsten Seitenasts und der Seitenast-Ansatz des diesem in distale Richtung nachgeordneten Seitenasts, bezogen auf einen jeweiligen Mittelpunkt der Seitenast-Ansätze, in Längsrichtung zwischen ca. 2 und 6 mm, vorzugsweise zwischen ca. 3,5 und 4,5 mm voneinander beabstandet sind.

Im Rahmen der Entwicklung der vorliegenden Erfindung wurde herausgefunden, dass mit den o.a. bevorzugten Werten für die Umfangswinkel sowie die Beabstandung der Seitenast-Ansätze voneinander eine Gefäßprothese geschaffen werden konnte, die insbesondere für die Aorta im Bereich der Abgänge des Truncus coealiacus, der Arteria mesenterica superior, sowie der rechten und linken Nierenarterien (Arteria renalis dextra und Arteria renalis sinistra) als off-the-shelf-Produkt geeignet ist. Dabei wird der am proximalsten angeordnete Seitenast dem Truncus coeliacus zugeordnet, der diesem in distale Richtung folgende Seitenast der Arteria mesenterica superior, und die beiden am distalsten angeordneten Seitenäste der rechten bzw. linken Nierenarterie.

In einer Weiterbildung der erfindungsgemäßen Gefäßprothese weist der proximale Endabschnitt einen größeren Durchmesser als der distale Endabschnitt auf, wobei gemäß einer Weiterbildung bevorzugt ist, wenn der proximale Endabschnitt einen sich verjüngenden Abschnitt in Übergang zum mittleren Abschnitt aufweist. Ferner kann der distale Endabschnitt im Übergang zum mittleren Abschnitt einen sich zum mittleren Abschnitt hin verjüngenden Abschnitt aufweisen.

Gemäß einer bevorzugten Ausführungsform weist der proximale Endabschnitt eine Länge von zwischen ca. 58 bis 70 mm, vorzugsweise von zwischen ca. 62 bis 66 mm auf, der sich verjüngende Abschnitt des proximalen Abschnitts eine Länge von ca. 20 bis 32 mm, vorzugsweise ca. 24 bis 28 mm, der mittlere Abschnitt eine Länge von ca. 56 bis 68 mm, vorzugsweise ca. 60 bis 64 mm, der sich zum mittleren Abschnitt hin verjüngende distale Abschnitt eine Länge von ca. 13 bis 25 mm, vorzugsweise von ca. 17 bis 21 mm, und der distale Endabschnitt eine Länge von ca. 28 bis ca. 40 mm, vorzugsweise von ca. 32 bis ca. 36 mm aufweisen. Die Gesamtlänge des proximalen Endabschnitts beträgt dabei ca. 86 bis ca. 96 mm, vorzugsweise ca. 88 bis 92 mm, die Gesamtlänge des distalen Endabschnitts ca. 40 bis 52 mm, vorzugsweise 44 bis 48 mm.

Die Gesamtlänge der Gefäßprothese beträgt vorzugsweise zwischen ca. 190 und 220 mm, vorzugsweise zwischen ca. 200 und 210 mm, und noch bevorzugter ca. 205 mm.

In einer weiteren Ausführungsform ist bevorzugt, wenn der Durchmesser der Seitenast-Lumen mit den ersten und zweiten proximalen Seitenast-Ansätzen von zwischen ca. 7 und 9 mm, vorzugsweise 8 mm beträgt.

Gemäß einer weiteren Ausführungsform der erfindungsgenäßen Gefäßprothese ist bevorzugt, wenn der Durchmesser der Seitenast-Lumen mit den ersten und zweiten distalen Seitenast-Ansätzen jeweils zwischen ca. 5 und 7 mm, vorzugsweise 6 mm beträgt.

Dabei ist ferner, in einer weiteren Ausführungsform bevorzugt, wenn der größte Durchmesser der im Wesentlichen ovalen Grundform der ersten Seitenast-Öffnungen, von zwischen ca. 12 bis 18 mm, vorzugsweise ca. 15 mm beträgt.

In einer Weiterbildung der erfindungsgemäßen Gefäßprothese sind zumindest im Bereich deren Enden und/oder der Seitenast-Enden und/oder im Bereich des Abgangs/der Öffnungen der nach innen abzweigenden Seitenäste röntgendichte Marker vorgesehen.

Vorzugsweise sind die röntgendichten Marker aus einem oder mehreren der folgenden Materialien, bspw. Gold, Palladium, Tantal, Chrom, Silber, etc.; die Form der Marker kann dabei beliebig sein, bspw. rund, eckig, und/oder bspw. die Form von Buchstaben, Zahlen oder Figuren haben, die für die Orientierung der Prothese im Gefäß hilfreich sind.

In einer Ausführungsform der erfindungsgemäßen Gefäßprothese ist der am proximalen Ende der Gefäßprothese angebrachte Stentring nur über seine nach distal weisenden Spitzbögen, nicht aber seine nach proximal weisenden Spitzbögen am Prothesenmaterial befestigt.

Gemäß einer weiteren Ausführungsform können die proximalen und distalen Abschnitte Stentringe mit unterschiedlichen, d.h. mit unterschiedlicher Amplitude in die distale und proximale Richtung ausschlagende Spitzbögen aufweisen. Auch die Seitenäste können in einer bevorzugten Ausführungsform zumindest einen, vorzugsweise einen, Stentring mit unterschiedlicher Amplitude der Spitzbögen aufweisen.

Weitere Vorteile ergeben sich aus den Figuren und der nachstehenden Beschreibung bevorzugter Ausführungsbeispiele.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:

- Fig. 1: eine schematische Darstellung einer Ausführungsform einer erfindungsgemäßen Gefäßprothese, im nicht-eingeführten, expandierten Zustand in perspektivischer Ansicht der Längsseite von seitlich-oben;
- Fig. 2: die Ausführungsform aus Fig. 1 in vollständig seitlicher Ansicht der Längsseite, mit schematischer Einzeichnung der Stenteringe und Seitenästen; von einer Seite (A), und in Ansicht einer weiteren Seite (B);
- Fig. 3: die Darstellung eines isolierten Seitenastes, in Ansicht auf die erste Seitenast-Öffnung von oben (A), bzw. von der Seite (B);
- Fig. 4: eine weitere schematische Darstellung einer Ausführungsform einer erfindungsgemäßen Gefäßprothese, wobei hier die Stentringe der besseren Übersicht wegen nicht eingezeichnet sind; in Ansicht von einer Seite (A); einer anderen Seite (B), sowie in Ansicht mit Blick in das Hauptlumen (C).

In den Figuren werden gleiche Merkmale mit gleichen Bezugszeichen versehen, wobei aus Gründen der Übersichtlichkeit nicht in allen Figuren immer sämtliche Bezugszeichen angegeben sind.

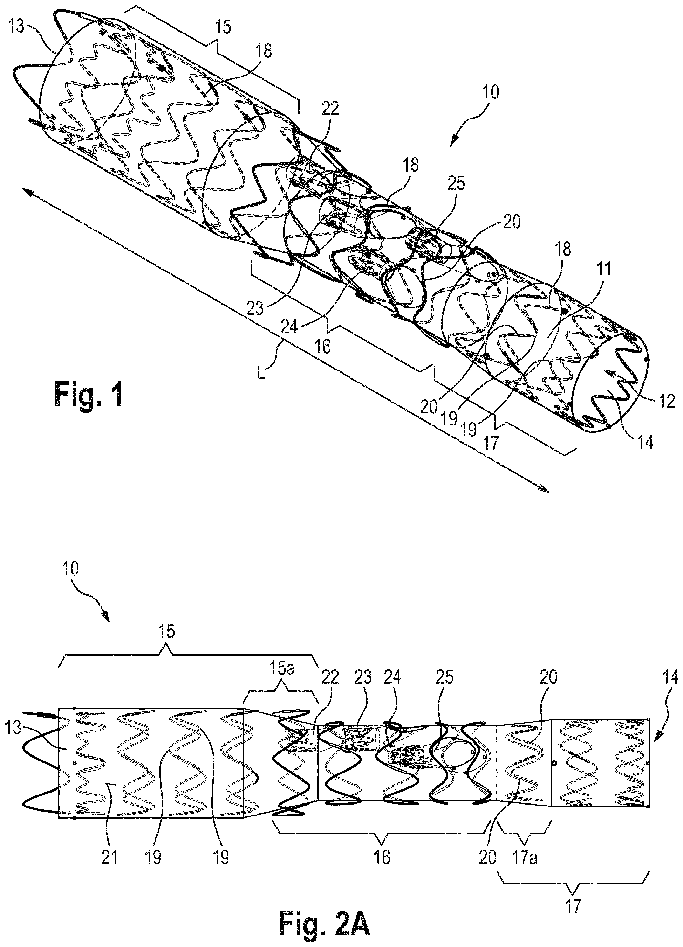

In den Figuren 1 bis 4 ist mit 10 insgesamt eine Gefäßprothese bezeichnet, mit einem generell hohlzylindrischen Grundkörper 11 mit einer Längsachse L, einer proximalen Öffnung 13 und einer distalen Öffnung 14. Die Gefäßprothese 10 weist ferner ein sich zwischen der proximalen und der distalen Öffnung 13,14 erstreckendes Hauptlumen 12 auf. Der Grundkörper 11 umfasst einen proximalen Endabschnitt 15 und einen distalen Endabschnitt 17 auf, sowie einen dazwischen angeordneten mittleren Abschnitt 16. Der proximale Endabschnitt 15 umfasst dabei die proximale Öffnung 13, der distale Endabschnitt 17 die distale Öffnung 14.

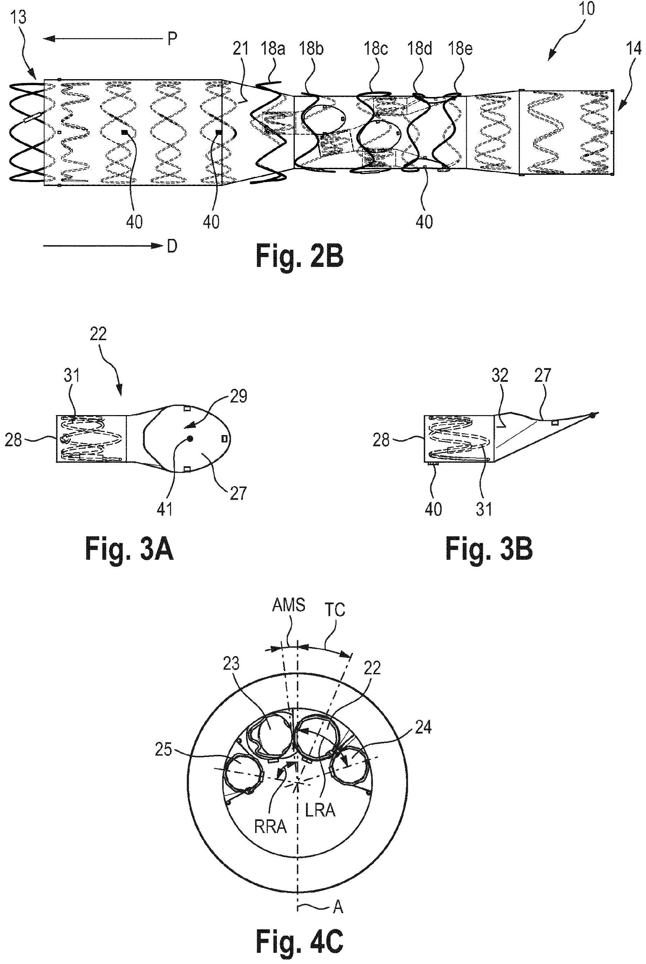

Wie Fig. 1 und Fig. 2 zu entnehmen ist, umfasst die Gefäßprothese 10 eine Vielzahl an einzelnen, nicht untereinander verbundenen Stentringen 18. Dies Stentringe 18 sind einstückig, d.h. geschlossen ringförmig, und mäanderförmig umlaufend ausgebildet. Die Stentringe 18 sind ferner über die Längsachse L der Gefäßprothese 10 hintereinander in einem Abstand von einander angeordnet, derart, dass sie sich nicht berühren. Die Stentringe 18 sind aus abwechselnd in die proximale und distale Richtung weisenden Spitzbögen 19 und diese verbindenden Stegen 20 gebildet.

Die Stentringe 18 sind an ein Prothesenmaterial 21 angebracht, und dadurch indirekt miteinander verbunden.

In Fig. 2B ist mit dem Pfeil "D" die distale Richtung angezeigt, und mit dem Pfeil "P" die proximale Richtung

Der mittlere Abschnitt 16 weist ferner einen Durchmesser d2 auf, der kleiner ist als die Durchmesser d1 und d3 der Endabschnitte 15 und 17.

Wie insbesondere Fig. 2 zu entnehmen ist, umfasst der proximale Endabschnitt 15 einen sich zum mittleren Abschnitt 16 hin verjüngenden Abschnitt 15a, und der distale Endabschnitt 17 einen sich zum mittleren Abschnitt 16 hin verjüngenden Abschnitt 17a. Die Verjüngung dieser Abschnitt ist in dem in Fig. 1 und 2 gezeigten Beispiel durch Stentringe 18 mit kleinerem Durchmesser als die Stentringe 18 der jeweiligen Endabschnitte 15, 17 bewirkt. Der Durchmesser des proximalen Endabschnitts 15 ist dabei größer als der Durchmesser des distalen Endabschnitts 17, wobei der mittlere Abschnitt 16 den kleinsten Durchmesser aufweist. Die Durchmesser der sich verjüngenden Abschnitte 15a, 17a des proximalen und des distalen Abschnitts 15, 17, verjüngen sich dabei kontinuierlich zum mittleren Abschnitt hin.

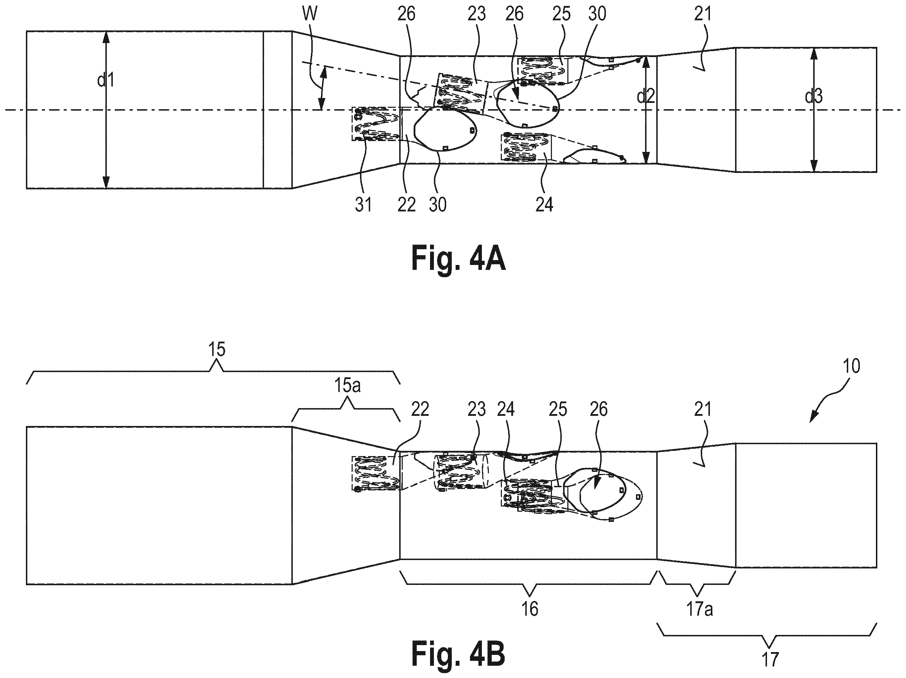

Den Figuren ist ferner zu entnehmen, dass der mittlere Abschnitt 16 vier Seitenäste 22, 23, 24, 25 umfasst, die nach innen, also ins Hauptlumen 12, abgehen. Die vier Seitenäste 22, 23, 24 und 25 erstrecken sich vom im Prothesenmaterial vorliegenden Fenestrierungen 26 in Richtung distal in das und im Hauptlumen 12 (siehe Fig. 4A, 4B).

Die Seitenäste 22, 23, 24 und 25 umfassen jeweils eine erste und eine zweite Seitenast-Öffnung 27, 28 (siehe Fig. 4A und 3B), sowie eine sich zwischen diesen Öffnungen 27, 28 erstreckendes Seitenast-Lumen 29. Die erste Öffnung 27 ist dabei fest an das Prothesenmaterial 21 des Grundkörpers 11 angebracht, und zwar über die Fenestrierung 26, zur Ausbildung eines Seitenast-Ansatzes 30. Die zweite Öffnung 28 ist frei im Hauptlumen 12.

Die Seitenäste 22, 23, 24 und 25 weisen jeweils einen Stentring 31 auf, der im Bereich der zweiten Öffnung 28 vorgesehen ist, wodurch diese Öffnung offen gehalten wird. Der Stentring 31 bildet zusammen mit einem Prothesenmaterial 32 (siehe Fig. 3) den Seitenast 22, 23, 24, und 25.

Wie insbesondere Fig. 3A und 3B zu entnehmen ist, in denen der Seitenast 22 im Detail gezeigt ist, und der im Aufbau den Seitenästen 23, 24, und 25 gleicht, weist die erste Seitenast-Öffnung 27 eine im Wesentlichen ovale Grundform auf, wohingegen die zweite Seitenast-Öffnung 28 eine im Wesentlichen runde Öffnung aufweist, bewirkt durch den ringförmigen Stentring 31.

Den Figuren ist ferner zu entnehmen, dass die vier Seitenäste 22, 23, 24 und 25 derart angeordnet sind, dass sich die beiden Seitenäste 22 und 23 proximal zu den Seitenästen 24 und 25 befinden, wobei der Seitenast 22 am proximalsten, und der Seitenast 25 am distalsten angeordnet ist. Fig. 4A ist dabei zu entnehmen, dass der proximalen Seitenast 23 bzw. dessen Seitenast-Ansatz 30, der dem proximalsten Seitenast 22 in distale Richtung nachangeordnet ist, in Bezug auf die Längsachse L der Gefäßprothese 10 verdreht angesetzt ist, was in Fig. 4A durch den Winkel W angezeigt ist. Die anderen Seitenastansätze 30 der Seitenäste 22, 24 und 25 sind parallel zur Längsachse L der Gefäßprothese angesetzt. Dabei ist den Figuren zu entnehmen, dass eine "Verdrehung" bedeutet, dass die ovale Grundform der Seitenast-Öffnung 29, die in unverdrehter Anordnung mit ihrem längeren Durchmesser parallel zur Längsachse L der Gefäßprothese angeordnet ist, in Bezug auf ihren längeren Durchmesser und in Bezug auf die Längsachse L der Gefäßprothese in einem bestimmten Winkel, vorzugsweise 8°, 9°, 10°, 11° oder 12° verdreht ist.

Wie den Figuren 1 und 2 ferner zu entnehmen ist, sind die Seitenast-Ansätze 30 der proximalen Seitenäste 22 und 23 im mittleren Abschnitt 16 nicht zwischen den gleichen Stentringen 18 angeordnet, wohingegen die Seitenast-Ansätze 30 der distalen Seitenäste 24 und 25 zwischen denselben Stentringen 18 angeordnet sind, wobei letztere etwas versetzt sind in Bezug auf den Umfangswinkel der Gefäßprothese 10.

Der mittlere Abschnitt 16 weist in dem in den Figuren 1 und 2 gezeigten Beispiel 5 Stentringe 18a bis e auf, die hintereinander von proximal nach distal angeordnet sind. Der Seitenast-Ansatz 30 des proximalsten Seitenasts 22 ist dabei zwischen den Stentringen 18b und 18c angeordnet, der Seitenast-Ansatz 30 des Seitenasts 23 zwischen den Stentringen 18c und 18d, und die beiden Seitenast-Ansätze 30 der Seitenäste 24, 25 zwischen den Stentringen 18d und 18e.

Die Seitenast-Ansätze 30 der Seitenäste 22, 23, 24, 25 sind über den Umfang bzw. die Mantelfläche des mittleren Abschnitts 16 der Gefäßprothese 10 versetzt zueinander angeordnet, so dass sich kein Seitenast 22, 23, 24, 25 entlang der Längsachse L der Gefäßprothese in einer gleichen gedachten Linie befindet.

Diese versetzte, im Umfangswinkel unterschiedliche Anordnung der Seitenast-Ansätze 30 der Seitenäste 22, 23, 24, 25 ist insbesondere in Fig. 4C gezeigt: hier ist mit der Linie A eine senkrechte Achse durch den Querschnitt der Gefäßprothese gezeigt, und zwar von proximaler Blickrichtung in die Gefäßprothese 10. Hier ist zu erkennen, dass der Seitenast 22 bzw. dessen Seitenast-Ansatz 30 in einem Winkel TC, vorzugsweise um 10°, verschoben ist, und dass der Seitenast 23, bzw. dessen Seitenast-Ansatz 30 um einen Winkel AMS, vorzugsweise 8°, verdreht ist, so dass die Seitenäste 22, 23, 24, 25 vollständig geöffnet sind, und sich nicht gegenseitig bedecken, und die Öffnungen dadurch verkleinern. Mit den Umfangswinkeln RRA und LRA ist angegeben, wie die beiden distalen Seitenast-Ansätze 30 der Seitenäste 24, 25 im Verhältnis zum Ansatz 30 des Seitenastes 23 winklig angeordnet sind.

In dem in den Figuren 1, 2 und 3 gezeigten Beispiel sind ferner Röntgenmarker angebracht, die in Fig. 2 durch die Pfeile 40 angezeigt sind. Wie insbesondere Fig. 2B zu entnehmen ist, sind entlang der Längsachse L der Gefäßprothese Marker 40 angebracht, sowie im Bereich der Seitenast-Ansätze 30 der Seitenäste 22, 23, 24, 25. Ferner befinden sich im Bereich der zweiten Öffnung der Seitenäste 22, 23, 24, und 25 Marker 40, was Fig. 3 zu entnehmen ist.

Die Länge der Gefäßprothese beträgt in dem in den Figuren 1, 2, und 3 gezeigten Beispiel ca. 205 mm.

Der Umfangsabstand der Ansätze 30 der Seitenäste 22, 23, 24, und 25 ist in dem in Fig. 1 bis 3 gezeigten Beispiel wie folgt: der Ansatz 30 des proximalsten Seitenasts 22 ist - bezogen auf einen Mittelpunkt 41 der Ansätze - vom Ansatz 30 des Seitenasts 23, der distal vom Ansatz 30 des Seitenasts 22 liegt, vorzugweise 20 mm des Seitenasts 22 beabstandet; ferner ist der Ansatz 30 des Seitenasts 23 vom Ansatz 30 des Seitenasts 24, der proximal zum Ansatz 30 des distalsten Seitenasts 25 liegt, bevorzugt 16 mm beabstandet; und der Ansatz 30 des Seitenasts 24 vom Ansatz 30 des distalsten Seitenasts 25 bevorzugt 4 mm beabstandet. Dabei bedeutet der damit angegebene Abstand den Abstand über die Längsachse gemessen, wobei, wie oben erwähnt, die Ansätze 30 der Seitenäste 22, 23, 24, und 25 im Umfang versetzt sind.

Vorteilhafterweise wird die erfindungsgemäße Gefäßprothese 10 zur Behandlung eines thorakal-abdominellen Aorten-Aneurysmas eines humanen Patienten eingesetzt, insbesondere im Bereich der Abgänge von Truncus coeliacus, der Arteria mesenterica superior und der beiden rechten und linken Nierenarterien. Dabei ist der Seitenast 22 für den Truncus coeliacus, der Seitenast 23 für die Arteria mesenterica superior, und die beiden distalen Seitenäste 24, 25, für die Arteria renalis dextra bzw. Arteria renalis sinistra vorgesehen bzw. ausgebildet.

## Patentansprüche

1. Gefäßprothese (10) für ein Blutgefäß eines Patienten, die folgendes aufweist:
einen hohlzylindrischen Grundkörper (11) mit einer Längsachse (L), einer proximalen (13) und einer distalen (14) Öffnung, sowie einem sich zwischen der proximalen (13) und der distalen (14) Öffnung erstreckenden Hauptlumen (12), wobei der Grundkörper (11) einen proximalen (15) und einen distalen (17) Endabschnitt aufweist, der jeweils die proximale (13) oder distale (14) Öffnung umfasst, sowie einem zwischen dem proximalen (15) und dem distalen (17) Endabschnitt angeordneten mittleren Abschnitt (16),
eine Vielzahl an nicht untereinander verbundenen Stentringen (18), die in Längsrichtung der Gefäßprothese (10) in einem Abstand hintereinander angeordnet und mäanderförmig umlaufend sind, wobei die Stentringe (18) jeweils Spitzbögen (19) und diese verbindenden Stege (20) aufweisen,
ein Prothesenmaterial (21), an welches die Stentringe (18) angebracht sind,
zumindest vier Seitenäste (22, 23, 24, 25), die sich von zwischen den Stentringen (18) liegenden Fenestrierungen (26) im Prothesenmaterial (21) ins Hauptlumen (12) nach distal erstrecken, wobei jeder Seitenast (22, 23, 24, 25) eine erste (27) und eine zweite (28) Seitenast-Öffnung sowie ein sich zwischen der ersten (27) und der zweiten (28) Seitenast-Öffnung erstreckendes Seitenast-Lumen (29) aufweist, wobei die erste Seitenast-Öffnung (27) jeweils fest an das Prothesenmaterial (21) über jeweils einer Fenestrierung (26) angebracht ist zur Ausbildung eines Seitenast-Ansatzes (30),
**dadurch gekennzeichnet, dass** die Gefäßprothese (10) vier Seitenäste (22, 23, 24, 25) aufweist, die sich ins Hauptlumen (12) erstrecken, und im mitterlen Abschnitt (16) angeordnet sind, wobei der mittlere Abschnitt (16) einen im Vergleich zu den Durchmessern d 1, d3 der Endabschnitte (15, 17) kleineren Durchmesser d2 aufweist.

2. Gefäßprothese (10) nach Anspruch 1, **dadurch gekennzeichnet, dass** die Stentringe (18) im mittleren Bereich (16) den gleichen Durchmesser aufweisen.

3. Gefäßprothese (10) nach einem der Ansprüche 1 oder 2, **dadurch gekennzeichnet, dass** sowohl der proximale Endabschnitt (15) als auch der distale (17) Endabschnitt jeweils einen sich zum mittleren Abschnitt (16) im Durchmesser verjüngenden Abschnitt (15a, 17a) aufweisen.

4. Gefäßprothese (10) nach einem der vorstehenden Ansprüche, **dadurch gekennzeichnet, dass** die erste Seitenast-Öffnung (27) eines jeden Seitenasts (22, 23, 24, 25), welche jeweils fest an das Prothesenmaterial (21) über jeweils einer Fenestrierung (26) angebracht ist, eine im Wesentlichen ovale Grundform aufweist.

5. Gefäßprothese (10) nach einem der vorstehenden Ansprüche, **dadurch gekennzeichnet, dass** die zweite Seitenast-Öffnung (28) eines jeden Seitenasts (22, 23, 24, 25) über eine im Wesentlichen kreisrunde Grundform aufweist.

6. Gefäßprothese (10) nach einem der vorstehenden Ansprüche, **dadurch gekennzeichnet, dass** die Seitenäste (22, 23, 24, 25) im mittleren Abschnitt (18) derart angeordnet sind, dass der Seitenast-Ansatz (30) eines ersten Seitenasts (23), in Bezug auf die Längsachse L des Grundkörpers (11), zwischen 5 und 15 °, vorzugsweise um 10° verdreht ist, derart, dass der Seitenast (23) in einem Winkel von 10° in Bezug auf die Längsachse L in das Hauptlumen (12) hineinragt, und dass die Seitenast-Ansätze (30) der drei anderen Seitenäste (22, 24, 25) in Bezug auf die Längsachse L unverdreht sind.

7. Gefäßprothese (10) nach einem der vorstehenden Ansprüche, **dadurch gekennzeichnet, dass** die Seitenäste (22, 23, 24, 25) verteilt und versetzt über den mittleren Abschnitt (16) angeordnet sind, derart, dass der Seitenast-Ansatz (30) des Seitenasts (24) und der Seitenast-Ansatz (30) des Seitenasts (25), die beide distal zu den anderen beiden Seitenästen (22, 23) angeordnet sind, zwischen denselben Stentringen (18d,18e) angeordnet sind, und der Seitenast-Ansatz (30) des am proximalsten angeordneten Seitenasts (22) und des diesem in distale Richtung nachgeordneten Seitenasts (23) zwischen zwei unterschiedlichen Stentringen (18b, 18c, 18d) angeordnet sind.

8. Gefäßprothese (10) nach Anspruch 7, **dadurch gekennzeichnet, dass** die Seitenast-Ansätze (30) der proximalsten Seitenäste (22, 23) in Umfangsrichtung des hohlzylindrischen Grundkörpers versetzt zueinander angeordnet sind.

9. Gefäßprothese (10) nach Anspruch 7 oder 8, **dadurch gekennzeichnet, dass** die Seitenast-Ansätze (30) der beiden proximalsten Seitenäste (22, 23) gegenüber den Seitenast-Ansätzen (30) der beiden distalsten Seitenäste (24, 25) in Umfangsrichtung versetzt zueinander angeordnet sind.

10. Gefäßprothese (10) nach einem der Ansprüche 7 bis 9, **dadurch gekennzeichnet, dass** der Seitenast-Ansatz (30) des proximalsten Seitenasts (22) und der Seitenast-Ansatz (30) des diesem in distale Richtung angeordneten Seitenasts (23) in einem Umfangswinkel von ca. 25° bis 35°, vorzugsweise von ca. 29° bis 32° versetzt zueinander angeordnet sind.

11. Gefäßprothese (10) nach einem der Ansprüche 7 bis 10, **dadurch gekennzeichnet, dass** der Seitenast-Ansatz (30) der beiden distalsten Seitenäste (24, 25) in einem Umfangswinkel von ca. 140° bis 160°, vorzugsweise von ca. 149° bis ca. 152° versetzte zueinander angeordnet sind.

12. Gefäßprothese (10) nach einem der Ansprüche 7 bis 11, **dadurch gekennzeichnet, dass** der Seitenast-Ansatz (30) des proximalsten Seitenasts (22) und der Seitenast-Ansatz (30) des diesem in distale Richtung nachgeordneten Seitenasts (23), bezogen auf einen jeweiligen Mittelpunkt (41) der Seitenast-Ansätze (30), in Längsrichtung zwischen ca. 15 und 25 mm, vorzugsweise zwischen ca. 18 und 22 mm voneinander beabstandet sind.

13. Gefäßprothese (10) nach einem der Ansprüche 7 bis 12, **dadurch gekennzeichnet, dass** der Seitenast-Ansatz (30) des Seitenasts (23) und der Seitenast-Ansatz (30) des diesem in distale Richtung nachgeordneten Seitenasts (24), bezogen auf einen jeweiligen Mittelpunkt (41) der Seitenast-Ansätze (30), in Längsrichtung zwischen ca. 12 und 20 mm, vorzugsweise zwischen ca. 14 und 18 mm, voneinander beabstandet sind.

14. Gefäßprothese (10) nach einem der Ansprüche 7 bis 13, **dadurch gekennzeichnet, dass** der Seitenast-Ansatz (30) des Seitenasts (24) und der Seitenast-Ansatz (30) des diesem in distale Richtung nachgeordneten Seitenasts (25), bezogen auf einen jeweiligen Mittelpunkt (41) der Seitenast-Ansätze (30), in Längsrichtung zwischen ca. 2 und 6 mm, vorzugsweise zwischen ca. 3,5 und 4,5 mm voneinander beabstandet sind.

15. Gefäßprothese (10) nach einem der Ansprüche 7 bis 14, **dadurch gekennzeichnet, dass** der Durchmesser der Seitenast-Lumen (20) der beiden am proximalsten angeordneten Seitenäste (22, 23) von zwischen ca. 7 und 9 mm, vorzugsweise 8 mm beträgt.

16. Gefäßprothese (10) nach einem der Ansprüche 7 bis 15, **dadurch gekennzeichnet, dass** der Durchmesser der Seitenast-Lumen (29) der beiden am distalsten angeordenten Seitenäste (24, 25) jeweils zwischen ca. 5 und 7 mm, vorzugsweise 6 mm beträgt.

17. Gefäßprothese (10) nach einem der Ansprüche 4 bis 16, **dadurch gekennzeichnet, dass** der größte Durchmesser der im Wesentlichen ovalen Grundform der ersten Seitenast-Öffnungen (27), von zwischen ca. 12 bis 18 mm, vorzugsweise ca. 15 mm beträgt.

## Claims

1. A vascular prosthesis (10) for a blood vessel of a patient, which has the following:
a hollow-cylindrical main body (11) with a longitudinal axis (L), a proximal opening (13) and a distal opening (14), and also a main lumen (12) extending between the proximal opening (13) and the distal opening (14), the main body (11) having a proximal end region (15) and a distal end region (17), which respectively comprise the proximal opening (13) or the distal opening (14), and also an intermediate region (16) arranged between the proximal end region (15) and the distal end region (17),
a plurality of stent rings (18), which are not connected to one another, which are arranged spaced apart one behind the other in the longitudinal direction of the vascular prosthesis (10) and running around in a meandering formation, the stent rings (18) respectively having ogives (19) and webs (20) connecting them,
a prosthesis material (21), to which the stent rings (18) are attached,
at least four side branches (22, 23, 24, 25), which extend distally into the main lumen (12) from fenestrations (26) in the prosthesis material (21) lying between the stent rings (18), wherein each side branch (22, 23, 24, 25) has a first side-branch opening (27) and a second side-branch opening (28) and also a side-branch lumen (29) extending between the first side-branch opening (27) and the second side-branch opening (28), the first side-branch opening (27) respectively being firmly attached to the prosthesis material (21), respectively, via a fenestration (26), such, that a side-branch attachment (30) is formed;
**characterized in that** the vascular prosthesis (10) has four side branches (22, 23, 24, 25), which extend into the main lumen (12), and are arranged in the intermediate region (16), the intermediate region (16) having a smaller diameter d2 in comparison with the diameters d1, d3 of the end regions (15, 17).

2. The vascular prosthesis (10) as claimed in claim 1, **characterized in that** the stent rings (18) in the intermediate region (16) have the same diameter.

3. The vascular prosthesis (10) as claimed in either of claims 1 and 2, **characterized in that** both the proximal end region (15) and the distal end region (17) each have a region (15a, 17a) tapering in diameter to the intermediate region (16).

4. The vascular prosthesis (10) as claimed in one of the preceding claims, **characterized in that** the first side-branch opening (27) of each side branch (22, 23, 24, 25), each of which is firmly attached to the prosthesis material (21) via a fenestration (26) respectively, has a substantially oval basic form.

5. The vascular prosthesis (10) as claimed in one of the preceding claims, **characterized in that** the second side-branch opening (28) of each side branch (22, 23, 24, 25) has a substantially circular basic form.

6. The vascular prosthesis (10) as claimed in one of the preceding claims, **characterized in that** the side branches (22, 23, 24, 25) in the intermediate region (16) are arranged in such a way that the side-branch attachment (30) of a first side branch (23) is turned, with respect to the longitudinal axis L of the main body (11), between 5 and 15°, preferably by 10°, such, that the side branch (23) protrudes into the main lumen (12) at an angle of 10° with respect to the longitudinal axis L, and **in that** the side-branch attachments (30) of the three other side branches (22, 24, 25) are unturned with respect to the longitudinal axis L.

7. The vascular prosthesis (10) as claimed in one of the preceding claims, **characterized in that** the side branches (22, 23, 24, 25) are arranged distributed and offset over the intermediate region (16), such, that the side-branch attachment (30) of the side branch (24) and the side-branch attachment (30) of the side branch (25), which are both distally arranged in relation to the other two side branches (22, 23), are arranged between the same stent rings (18d, 18e), and the side-branch attachment (30) of the most proximally arranged side branch (22) and of the side branch (23) arranged downstream thereof in the distal direction are arranged between two different stent rings (18b, 18c, 18d).

8. The vascular prosthesis (10) as claimed in claim 7, **characterized in that** the side-branch attachments (30) of the most proximal side branches (22, 23) are arranged offset in relation to one another in the circumferential direction of the hollow-cylindrical main body.

9. The vascular prosthesis (10) as claimed in claim 7 or 8, **characterized in that** the side-branch attachments (30) of the two most proximal side branches (22, 23) are arranged offset in relation to one another in the circumferential direction with respect to the side-branch attachments (30) of the two most distal side branches (24, 25).

10. The vascular prosthesis (10) as claimed in one of claims 7 to 9, **characterized in that** the side-branch attachment (30) of the most proximal side branch (22) and the side-branch attachment (30) of the side branch (23) arranged thereto in the distal direction are arranged offset in relation to one another at a circumferential angle of about 25° to 35°, preferably of about 29° to 32°.

11. The vascular prosthesis (10) as claimed in one of claims 7 to 10, **characterized in that** the side-branch attachment (30) of the two most distal side branches (24, 25) are arranged offset in relation to one another at a circumferential angle of about 140° to 160°, preferably of about 149° to about 152°.

12. The vascular prosthesis (10) as claimed in one of claims 7 to 11, **characterized in that** the side-branch attachment (30) of the most proximal side branch (22) and the side-branch attachment (30) of the side branch (23) downstream thereof in the distal direction are spaced apart from one another, with respect to a respective midpoint (41) of the side-branch attachments (30), in the longitudinal direction by between about 15 and 25 mm, preferably between about 18 and 22 mm.

13. The vascular prosthesis (10) as claimed in one of claims 7 to 12, **characterized in that** the side-branch attachment (30) of the side branch (23) and the side-branch attachment (30) of the side branch (24) arranged downstream thereof in the distal direction are spaced apart from one another, with respect to a respective midpoint (41) of the side-branch attachments (30), in the longitudinal direction by between about 12 and 20 mm, preferably between about 14 and 18 mm.

14. The vascular prosthesis (10) as claimed in one of claims 7 to 13, **characterized in that** the side branch attachment (30) of the side branch (24) and the side-branch attachment (30) of the side branch (25) arranged downstream thereof in the distal direction are spaced apart from one another, with respect to a respective midpoint (41) of the side-branch attachments (30), in the longitudinal direction by between about 2 and 6 mm, preferably between about 3.5 and 4.5 mm.

15. The vascular prosthesis (10) as claimed in one of claims 7 to 14, **characterized in that** the diameter of the side-branch lumen (20) of the two most proximally arranged side branches (22, 23) is of between about 7 and 9 mm, preferably 8 mm.

16. The vascular prosthesis (10) as claimed in one of claims 7 to 15, **characterized in that** the diameter of the side-branch lumen (29) of the two most distally arranged side branches (24, 25) is in each case between about 5 and 7 mm, preferably 6 mm.

17. The vascular prosthesis (10) as claimed in one of claims 4 to 16, **characterized in that** the greatest diameter of the substantially oval basic form of the first side-branch openings (27) is of between about 12 to 18 mm, preferably about 15 mm.

## Revendications

1. Prothèse vasculaire (10) pour un vaisseau sanguin d'un patient, présentant :
un corps de base (11) cylindrique creux ayant un axe longitudinal (L), une ouverture proximale (13) et une ouverture distale (14), ainsi qu'une lumière principale (12) s'étendant entre l'ouverture proximale (13) et l'ouverture distale (14), le corps de base (11) présentant une partie d'extrémité proximale (15) et une partie d'extrémité distale (17) qui comprennent respectivement l'ouverture proximale (13) ou l'ouverture distale (14), ainsi qu'une partie centrale (16) disposée entre la partie d'extrémité proximale (15) et la partie d'extrémité distale (17),
une pluralité d'anneaux de stent (18) non reliés entre eux, qui sont disposés à distance les uns après les autres dans la direction longitudinale de la prothèse vasculaire (10) et se présentent en forme de méandres périphériques, les anneaux de stent (18) présentant respectivement des ogives (19) et des entretoises (20) qui les relient,
un matériau de prothèse (21) sur lequel sont montés les anneaux de stent (18),
au moins quatre branches latérales (22, 23, 24, 25) qui s'étendent dans le sens distal dans la lumière principale (12) à partir de fenêtres (26) situées entre les anneaux de stent (18) dans le matériau de prothèse (21), chaque branche latérale (22, 23, 24, 25) présentant une première (27) et une deuxième (28) ouverture de branche latérale ainsi qu'une lumière de branche latérale (29) s'étendant entre la première (27) et la deuxième (28) ouverture de branche latérale, la première ouverture de branche latérale (27) étant respectivement montée solidement sur le matériau de prothèse (21) par l'intermédiaire d'une fenêtre (26) respectivement pour réaliser une saillie de branche latérale (30), **caractérisée en ce que** la prothèse vasculaire (10) présente quatre branches latérales (22, 23, 24, 25) qui s'étendent dans la lumière principale (12) et sont disposées dans la partie centrale (16), la partie centrale (16) présentant un diamètre d2 inférieur en comparaison avec les diamètres d1, d3 des parties d'extrémité (15, 17).

2. Prothèse vasculaire (10) selon la revendication 1, **caractérisée en ce que** les anneaux de stent (18) présentent le même diamètre dans la zone centrale (16).

3. Prothèse vasculaire (10) selon l'une quelconque des revendications 1 et 2, **caractérisée en ce qu'**à la fois la partie d'extrémité proximale (15) et la partie d'extrémité distale (17) présentent respectivement une partie (15a, 17a) dont le diamètre diminue en direction de la partie centrale (16).

4. Prothèse vasculaire (10) selon l'une quelconque des revendications précédentes, **caractérisée en ce que** la première ouverture de branche latérale (27) de chaque branche latérale (22, 23, 24, 25), qui est respectivement montée solidement sur le matériau de prothèse (21) par respectivement une fenêtre (26), présente une forme de base substantiellement ovale.

5. Prothèse vasculaire (10) selon l'une quelconque des revendications précédentes, **caractérisée en ce que** la deuxième ouverture de branche latérale (28) de chaque branche latérale (22, 23, 24, 25) présente une forme de base substantiellement circulaire.

6. Prothèse vasculaire (10) selon l'une quelconque des revendications précédentes, **caractérisée en ce que** les branches latérales (22, 23, 24, 25) dans la partie centrale (18) sont disposées de telle sorte que la saillie de branche latérale (30) d'une première branche latérale (23) est pivotée entre 5 et 15°, de préférence de 10°, par rapport à l'axe longitudinal L du corps de base (11) de telle sorte que la branche latérale (23) fait saillie dans la lumière principale (12) selon un angle de 10° par rapport à l'axe longitudinal L, et **en ce que** les saillies de branche latérale (30) des trois autres branches latérales (22, 24, 25) ne sont pas pivotées par rapport à l'axe longitudinal L.

7. Prothèse vasculaire (10) selon l'une quelconque des revendications précédentes, **caractérisée en ce que** les branches latérales (22, 23, 24, 25) sont disposées de manière répartie et décalée sur la partie centrale (16) de telle sorte que la saillie de branche latérale (30) de la branche latérale (24) et la saillie de branche latérale (30) de la branche latérale (25), qui sont toutes les deux disposées de manière distale par rapport aux deux autres branches latérales (22, 23), sont disposées entre les mêmes anneaux de stent (18d, 18e), et la saillie de branche latérale (30) de la branche latérale (22) disposée de la manière la plus proximale et de la branche latérale (23) placée en aval de celle-ci dans la direction distale sont disposées entre deux anneaux de stent (18b, 18c, 18d) différents.

8. Prothèse vasculaire (10) selon la revendication 7, **caractérisée en ce que** les saillies de branche latérale (30) des branches latérales (22, 23) les plus proximales dans la direction circonférentielle du corps de base cylindrique creux sont disposées de manière décalée l'une par rapport à l'autre.

9. Prothèse vasculaire (10) selon la revendication 7 ou 8, **caractérisée en ce que** les saillies de branche latérale (30) des deux branches latérales (22, 23) les plus proximales sont disposées de manière décalée l'une par rapport à l'autre dans la direction circonférentielle, à l'opposé des saillies de branche latérale (30) des deux branches latérales (24, 25) les plus distales.

10. Prothèse vasculaire (10) selon l'une quelconque des revendications 7 à 9, **caractérisée en ce que** la saillie de branche latérale (30) de la branche latérale (22) la plus proximale et la saillie de branche latérale (30) de la branche latérale (23) disposée dans la direction distale sont disposées de manière décalée l'une par rapport à l'autre selon un angle circonférentiel d'environ 25° à 35°, de préférence d'environ 29° à 32°.

11. Prothèse vasculaire (10) selon l'une quelconque des revendications 7 à 10, **caractérisée en ce que** les saillies de branche latérale (30) des deux branches latérales (24, 25) les plus distales sont disposées de manière décalée l'une par rapport à l'autre selon un angle circonférentiel d'environ 140° à 160°, de préférence d'environ 149° à environ 152°.

12. Prothèse vasculaire (10) selon l'une quelconque des revendications 7 à 11, **caractérisée en ce que** la saillie de branche latérale (30) de la branche latérale (22) la plus proximale et la saillie de branche latérale (30) de la branche latérale (23) placée en aval de celle-ci dans la direction distale sont espacées l'une par rapport à l'autre dans la direction longitudinale entre 15 et 25 mm environ, de préférence entre 18 et 22 mm environ, par rapport à un centre respectif (41) des saillies de branche latérale (30).

13. Prothèse vasculaire (10) selon l'une quelconque des revendications 7 à 12, **caractérisée en ce que** la saillie de branche latérale (30) de la branche latérale (23) et la saillie de branche latérale (30) de la branche latérale (24) placée en aval de celle-ci dans la direction distale sont espacées l'une par rapport à l'autre dans la direction longitudinale entre 12 et 20 mm environ, de préférence entre 14 et 18 mm environ, par rapport à un centre respectif (41) des saillies de branche latérale (30).

14. Prothèse vasculaire (10) selon l'une quelconque des revendications 7 à 13, **caractérisée en ce que** la saillie de branche latérale (30) de la branche latérale (24) et la saillie de branche latérale (30) de la branche latérale (25) placée en aval de celle-ci dans la direction distale sont espacées l'une par rapport à l'autre dans la direction longitudinale entre 2 et 6 mm environ, de préférence entre 3,5 et 4,5 mm environ, par rapport à un centre respectif (41) des saillies de branche latérale (30).

15. Prothèse vasculaire (10) selon l'une quelconque des revendications 7 à 14, **caractérisée en ce que** le diamètre des lumières de branche latérale (20) des deux branches latérales (22, 23) disposées de la manière la plus proximale est compris entre 7 et 9 mm environ, de préférence égal à 8 mm.

16. Prothèse vasculaire (10) selon l'une quelconque des revendications 7 à 15, **caractérisée en ce que** le diamètre des lumières de branche latérale (29) des deux branches latérales (24, 25) disposées de la manière la plus distale est compris respectivement entre 5 et 7 mm environ, de préférence égal à 6 mm.

17. Prothèse vasculaire (10) selon l'une quelconque des revendications 4 à 16, **caractérisée en ce que** le plus grand diamètre de la forme de base substantiellement ovale des premières ouvertures de branche latérale (27) est compris entre 12 et 18 mm environ, de préférence égal à 15 mm.
